# EUROPEAN PATENT SPECIFICATION

(11) **EP 3 867 638 B1**
(45) Date of publication and mention of the grant of the patent: **04.12.2024**
(21) Application number: 19872882.6
(22) Date of filing: 17.10.2019
(51) Int. Cl.: G01N 33/49, G01N 15/10, B01D 15/26, B01J 20/20, B01J 20/28, A61K 35/14, G01N 15/00, G01N 33/50, G01N 33/574

(54) **ADSORPTION AND BINDING OF PLASMA MOLECULES AND PARTICLES TO CARBON**
ADSORPTION UND BINDUNG VON PLASMAMOLEKÜLEN UND PARTIKELN AN KOHLENSTOFF
ADSORPTION ET LIAISON DE MOLÉCULES ET DE PARTICULES DE PLASMA À DU CARBONE

(30) Priority: 17.10.2018 US 201862746694 P
(43) Date of publication of application: 25.08.2021
(73) Proprietor: Immutrix Therapeutics, Inc., Rapid City, South Dakota 57703 (US)
(72) Inventor: DIAZ-AUNON, Jose A., Rapid City, South Dakota 57703 (US); SANDIFORD, Oleta A., Newark, New Jersey 07103 (US); RAMESHWAR, Pranela, Newark, New Jersey 07103 (US)
(74) Representative: D Young & Co LLP
(86) International application number: PCT/US2019/056816
(87) International publication number: WO 2020/081865

(56) References cited:
- WO-A2-2007/103572
- WO-A2-2007/103572
- KR-B1- 101 839 347
- US-A1- 2013 072 845
- US-A1- 2013 196 355
- US-A1- 2013 323 756
- US-A1- 2017 232 183
- US-A1- 2017 232 183

## Description

### TECHNICAL FIELD

The present invention relates to methods for quantifying an amount of exosomes in whole blood derived from a subject as compared to controls and in order to diagnose a medical condition.

### BACKGROUND

Exosomes are small nanoparticles that are typically about 50 nm to about 120 nm in size with an internal cargo that may comprise for example DNA, RNA, proteins and lipids. These extracellular vesicles are known to be crucial to intercellular and long-distance communication. Exosomes are therefore released from both healthy cells and disease cells, such as but not limited to cancer cells.

It has previously been found that cancer cells release exosomes which are involved in: mediating distant metastasis, establishing a metastatic niche and influencing the microenvironment to support tumor growth. A need therefore exists to reduce the amount of exosomes present in the blood of patients suffering from a disease state. Limiting the amount of such exosomes would for example impede cellular communication and limit the progression of a disease. Thus, the disclosure herein addresses such needs and provides both a method of diagnosing a medical condition in a subject by quantifying the amount of exosomes in the blood of the subject, and a method of treating such a medical condition by reducing the quantity of exosomes in the whole blood or plasma of a subject. Such a method utilizes a synthetic carbon particle (SCP) as an adsorbent material.

WO 2007/103572 relates to extra corporeal removal of microvesicular particles.

### BRIEF DESCRIPTION OF THE DRAWINGS

For a more complete understanding of the present disclosure, reference will now be made to the accompanying drawings/figures in which:
Figure 1(A) depicts a graphical representation of the number of exosomal nanoparticles that were unbound in control and patient plasma pre surgery as disclosed in an embodiment described herein;
Figure 1(B) and Figure 1(C) depict a graphical representation of the number of exosomal nanoparticles that were unbound in two post-surgery plasma samples (of September 18, 2018, and October 3, 2018 respectively) as disclosed in an embodiment described herein;

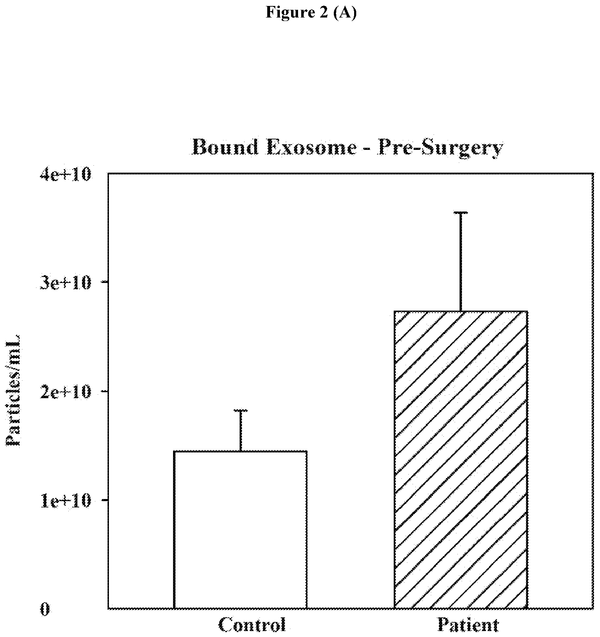

Figure 2(A) depicts a graphical representation of the number of exosomal nanoparticles that were bound in control and patient plasma collected and measured pre surgery as disclosed in an embodiment described herein;

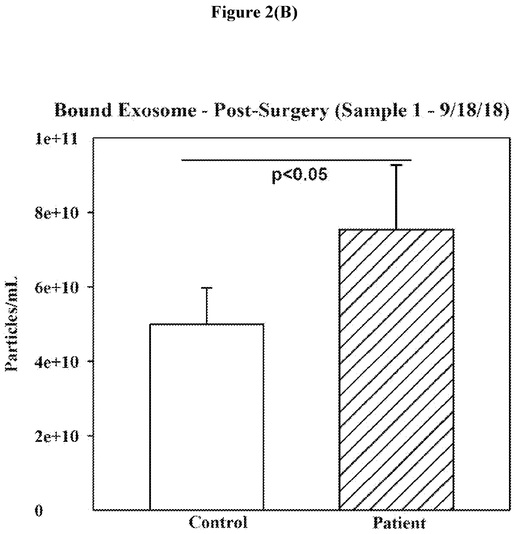

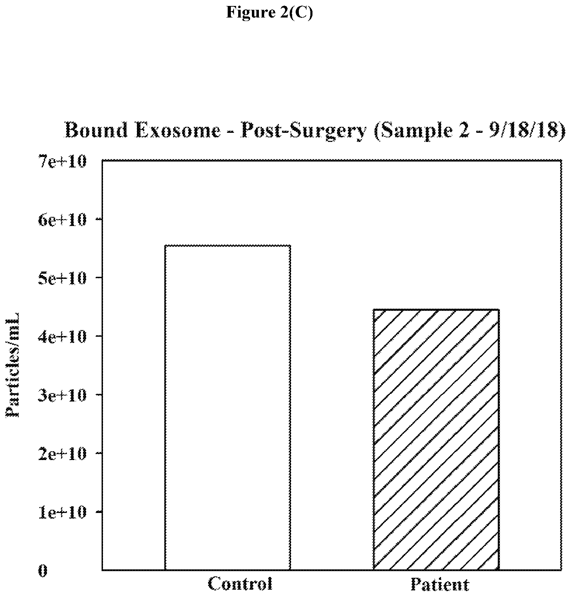

Figure 2 (B) and Figure 2(C) depict a graphical representation of the number of exosomal nanoparticles that were bound in two post-surgery plasma samples as disclosed in an embodiment described herein;
Figure 3 depicts a gel electrophoresis of bound and unbound samples from Experiment 1 as described herein, which were electrophoresed on 2% agarose in order to identify the presence of cell free DNA;
Figure 4 depicts a graph of a Bradford assay for the total amount of protein assessed in the bound and unbound samples from Experiment 1 as described herein and compared to the control;
Figure 5 depicts a prior art electron microscopic image of an endocytosis process whereby exosomes are released from a cell *in vitro*;
Figure 6 (A-C): (A) depicts the characterized of endosome size by nanosight tracking analysis (NTA), wherein the data shows that the exosomes are within a range of 50-100 nm; (B) depicts a tabulation of the size of tested SCPs; and (C) depicts binding data (number of particles) of purified exosomes to carbon SCP-1 and SCP-3; as disclosed by an embodiment described herein;
Figure 7 (A) depicts a graphical representation of the number of exosomes bound to different SCPs, wherein SCP-1 adsorbs the most exosomes, in an embodiment disclosed herein; Figure 7(B) depicts an nanosight tracking analysis (NTA) which validates that the particles trapped in the SCPs were exosomes as disclosed herein; and
Figure 8 (A) depicts a graphical representation of binding data for carbon SCP-1, confirming that SCP-1 can bind/adsorb exosomes from the plasma of a sixty year old ovarian cancer patient, and plasma from a healthy donor of similar age (left); and Figures 8(B) and(C) graphically depict further binding studies providing confirmation that the exosomes of embodiments disclosed herein bind to the carbon as characterized by exosome specific markers.

### SUMMARY OF THE DISCLOSURE

The present invention provides a method for quantifying an amount of exosomes in whole blood which comprises obtaining whole blood from a subject; separating the whole blood into plasma, and at least one cellular blood fraction, wherein the plasma comprises a first amount of unbound exosomes; contacting the plasma with an adsorbent material comprising a synthetic carbon particle (SCP), and forming treated plasma, and a complex, wherein the treated plasma comprises a second amount of unbound exosomes, and the complex comprises an amount of SCP-bound-exosomes; and comparing the second amount of unbound exosomes with a first control and/or comparing the SCP-bound-exosomes with a second control, wherein the SCP comprises mesopores, micropores, macropores, or a combination thereof, further wherein the mesopores are 2 nm to 50 nm in diameter, wherein the micropores are less than 2 nm in diameter, and wherein the macropores are 50 to 120 nm in diameter. In some embodiments of the method, the first control comprises a known amount of unbound exosomes, and the second control comprises a known amount of bound-exosomes.

In a further embodiment of the method, the subject is diagnosed with or suspected of having a medical condition when the second amount of unbound exosomes are different from the first control and/or when the amount of SCP-bound-exosomes are different from the second control.

The medical condition may be a cancer, wherein the cancer may be selected from the group consisting of pancreatic cancer, ovarian cancer, glioblastoma, and combinations thereof.

In the method according to the invention, the SCP comprises mesopores, micropores, macropores, or a combination thereof, wherein the mesopores are 2 nm to 50 nm in diameter, the micropores are less than 2 nm in diameter, and the macropores are 50 to 120 nm in diameter.

In some embodiments of the method, the amount of bound exosomes is from about 10² exosomes/g SCP to equal to or greater than about 10⁹ exosomes/g SCP, in some further embodiment the synthetic carbon particle (SCP) comprises a surface area of about 300-900 m²/g.

In a further embodiment of the method according to the invention, the adsorbent material comprises a synthetic carbon particle (SCP), wherein the synthetic carbon particle (SCP) is at least one of SCP-1, SCP-2, SCP-3, SCP-4, or a combination thereof; in a still further embodiment the synthetic carbon particle (SCP) is SCP-1. In some embodiments of the method the SCP-1 comprises a surface area of about 300-900 m²/g.

The foregoing has outlined rather broadly certain of the features of the exemplary embodiments of the present invention in order that the detailed description that follows may be better understood. It should be appreciated by those skilled in the art that the conception and the specific embodiments disclosed may be readily utilized as a basis for modifying or designing other methods and structures for carrying out the same purposes of the invention that is claimed below.

### DETAILED DESCRIPTION OF DISCLOSED EXEMPLARY EMBODIMENTS

It should be understood at the outset that although an illustrative implementation of one or more embodiments are provided below, the disclosed systems and/or methods may be implemented using any number of techniques, whether currently known or in existence.

The drawing figures are not necessarily to scale. Certain features and components herein may be shown exaggerated in scale or in somewhat schematic form and some details of conventional elements may be omitted in interest of clarity and conciseness.

In the following discussion and in the claims, the terms "including" and "comprising" are used in an open-ended fashion, and thus should be interpreted to mean "including, but not limited to...." Also, the term "couple" or "couples" is intended to mean either an indirect or direct connection. Thus, if a first component or device couples to a second, that connection may be through a direct engagement between the two components or devices, or through an indirect connection that is made via other intermediate devices and connections. As used herein, the term "about," when used in conjunction with a percentage or other numerical amount, means plus or minus 10% of that percentage or other numerical amount. For example, the term "about 80%," would encompass 80% plus or minus 8%. As used herein the terminology instrument, apparatus, and device may be used interchangeably. The term "subject," as used herein, comprises any and all organisms and includes the term "patient."

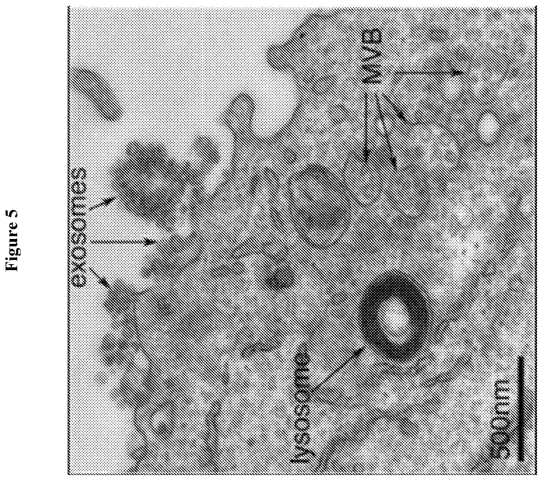

Mammalian cells can communicate (i.e., signal) via exosomes. Generally, exosomes are extracellular membrane-derived vesicles having in some embodiments a size of about 50 nm to about 120 nm in diameter. Exosomes, in some other embodiments may be considered to be cell derived nanoparticles that are comprises in all bodily fluids (*See* for example the formation of exosomes in Figure 5).

Further, exosomes may comprise various bioactive molecules, such as but not limited to: RNA, messenger RNA, long-noncoding RNA, microRNA, DNA, mtDNA enzymes, proteins (such as but not limited to signaling proteins), etc. or combinations thereof.

Exosomes carry information (e.g., function as signaling molecules) that can play a role in a medical condition; e.g., exosomes can interfere with treating or curing a medical condition, and can promote the worsening of a medical condition, etc. For example, exosomes can promote cancer tumor growth by reprogramming surrounding cells via the transfer of nucleic acids and proteins that activate specific signaling pathways. As another example, exosomes can prepare host organs for metastasis by remodeling the extracellular matrix and promoting angiogenesis in potential host organs.

Thus, without wishing to be limited by theory, the composition of exosomes derived from diseased cells (such as cancer cells) is often distinct from that of exosomes derived from healthy cells (i.e., non-diseased cells). Consequently, exosomes in the blood of a subject suffering from a medical condition may be different from the exosomes in the blood of a healthy subject. Exosomes are thus in some embodiments considered to be integral to neurodegenerative disorders, such as but not limited to Alzheimer's disease, and to the mechanisms involved in cancer metastasis.

In some embodiments, mesenchymal stem cell (MSC) derived exosomes may play a role in mechanisms associated with cell survival, drug resistance, and in some further embodiments induce cellular quiescence in breast cancer cells. In other embodiments exosomes may be utilized in drug delivery, such that they may not invoke an immune response in the subject.

In an embodiment, a blood sample is obtained from a subject who is in fluid communication with an extracorporeal apparatus (described herein below). The subject may have or may be suspected of having a medical condition or disease, and thus the whole blood or plasma may comprise disease derived exosomes.

In some embodiments, the extracorporeal apparatus (or in some embodiments an extracorporeal circuit) is an apparatus having columns containing adsorbent materials, as described above.

An embodiment of an apparatus suitable for use in the present disclosure is described in detail in U.S. Patent # 9,669,151 and may comprises an inlet in fluid communication with a pump which regulates access and fluid communication with a conduit. The apparatus may be connected to a subject through establishing a means of blood flow from the subject to the inlet. An arterial access of the subject may be used to establish a means of blood flow from the subject to the inlet.

An embodiment of a methodology of the type disclosed herein comprises establishing fluid communication between a subject's blood flow as accessed through a vein (e.g., jugular, subclavian or femoral veins) of the subject and the inlet of the apparatus. Alternatively the subject's blood flow may be accessed via one or more chronic vascular accesses. For example, a chronic vascular access may have been created by a surgical procedure: such as (i) native arteriovenous fistulas (native AVFs), (ii) arteriovenous shunts using graft material (AV graft), and (iii) tunneled double-lumen catheters. The pump regulates the flow of the subject's blood to the remainder of the apparatus through conduit, which may be a pipe or flow line comprised of material suitable for use in the methodologies disclosed herein.

In an embodiment, the subject's blood is allowed to flow through the conduit until it reaches a valve which when in the on position allows the blood flow to enter a column (A) in a particular flow direction. Blood may be pumped through the column and exit the column thorough a valve regulated outlet. Blood exiting from column A through the outlet may enter the conduit where it is pumped to an inlet port whose access is regulated by valve. When valve is in the on position, the blood may be pumped from inlet port to a second column (B). In an embodiment, the subject's blood is allowed to flow through another conduit, reaches inlet port and in some embodiments is allowed to enter column C. The blood may exit column C which is regulated by a further valve which when in the on position allows the blood to flow into a conduit and back to the vein of the subject.

Further, in an embodiment the rate of flow of a bodily fluid (e.g., blood) through an apparatus may be regulated to provide some user and/or process goal. For example, the rate of blood flow through apparatus may range from about 1 mL/min to about 300 mL/min, alternatively from about 25 mL/min to about 300 mL/min, alternatively from about 25 mL/min to about 150 mL/min, or alternatively from about 150 mL/min to about 300 mL/min.

Treatment of a subject suffering from but not limited to septic shock, cancer, glioblastoma multiform (GMB), and preeclampsia may require the subject be in fluid communication with the apparatus for a period of time ranging from about 1 hour to about 24 hours, alternatively from about 1 hour to about 12 hours, alternatively from about 1 hour to about 6 hours, alternatively from about 1 hour to about 4 hours, or alternatively less than about 4 hours. In an embodiment, the subject is in fluid communication with the apparatus for a time period sufficient to allow from about 0.5 to about 10 times the total blood volume of the subject to circulate through the apparatus, alternatively, from about 1 to about 10 times the total blood volume of the subject is allowed to circulate through the apparatus. In yet another embodiment, the blood volume circulated through the apparatus may range from about 5 liters to about 72 liters, alternatively from about 10 liters to about 60 liters, or alternatively from about 36 liters to about 54 liters and may occur in a time period ranging from about 1 hour to about 6 hours or alternatively from about 3 hours to about 4 hours. The subject may undergo treatments where they are placed in fluid communication with the apparatus a plurality of times as deemed sufficient to address their particular disease state.

In one embodiment, adsorbent materials are disposed within or contained by the columns (A), (B), and (C) of the apparatus. Adsorbent materials suitable for use in the present disclosure include chromatographic materials, which have been subjected to a sanitization process, such as selected from the group consisting of anion exchange resins, cation exchange resins, and combinations thereof.

The adsorbent materials used in the method of the present invention comprise synthetic carbon particle(s), wherein said particles comprise carbon beads. In some embodiments, SCPs herein effectively adsorb/bind exosomes. The SPCs may reduce cancer-derived exosomes in the circulation of a subject via contact with such materials. Such beads are described in detail in WO 20191126367 (PCT/US2018/066568).

The beads disclosed may be carbonaceous materials derived from polycondensation resinous materials whose porosity can be tailored such that they may efficiently adsorb exosomes as described herein.

The SCP may comprise a carbonaceous material which has a pore size (p) ranging from a lower limit (a) to an upper limit (z) and a bulk density (a) ranging from a lower limit (b) to an upper limit (y) where the comparative variability (g) defined as (y-b )/(z-a) is less than 1.Carbonaceous material may have a pore size ranging from about 10 nm to about 5000 nm and a bulk density ranging from 0.06 g/ml to 0.15 g/ml, or from about 20 nm to about 300 nm and a bulk density ranging from about 0.3 g/ml to about 0.5 g/ml, or from about 50 nm to about 150 nm and a bulk density ranging from about 0.3 g/ml to about 0.5 g/ml, although the SCP used in the method according to the invention comprises mesopores, micropores, macropores, or a combination thereof, wherein the mesopores are 2 nm to 50 nm in diameter, the micropores are less than 2 nm in diameter, and the macropores are 50 to 120 nm in diameter. Polycondensation resin may comprise a high-ortho phenol resin having a pore size ranging from about 10 nm to about 500 nm and an intraparticular density ranging from about 2% to about 25%. Polycondensation resin may have a pore size of from about 25 nm to about 300 nm and an intraparticular porosity ranging from about 5% to about 20%, or a pore size of from about 50 nm to about 150 nm and an intraparticular porosity ranging from about 8% to about 15%.

In some embodiments, the polycondensation resin may comprise a chelating agent. In some embodiments, a carbonaceous material has a pore size (p) ranging from a lower limit (a) to an upper limit (z) and a bulk density (a) ranging from a lower limit (b) to an upper limit (y) where the comparative variability (g) defined as (y-b )/(z-a) is less than 1 × 10-3. Carbonaceous material may have a pore size ranging from about 10 nm to about 5000 nm and a bulk density ranging from 0.06 g/ml to 0.15 g/ml, or a pore size ranging from about 20 nm to about 300 nm and a bulk density ranging from about 0.3 g/ml to about 0.5 g/ml, or a pore size ranging from about 50 nm to about 150 nm and a bulk density ranging from about 0.3 g/ml to about 0.5 g/ml, although the SCP used in the method according to the invention comprises mesopores, micropores, macropores, or a combination thereof, wherein the mesopores are 2 nm to 50 nm in diameter, the micropores are less than 2 nm in diameter, and the macropores are 50 to 120 nm in diameter. Carbonaceous material may comprise an adsorbent or a film. In some embodiments, a carbonaceous material has a pore size (p) ranging from a lower limit (a) to an upper limit (z) and a bulk density (a) ranging from a lower limit (b) to an upper limit (y) where the comparative variability (g) defined as (y-b)/(z-a) is less than 1 × 10-5. Carbonaceous material may have a pore size ranging from about 10 nm to about 5000 nm and a bulk density ranging from 0.06 g/ml to 0.15 g/ml, or a pore size ranging from about 20 nm to about 300 nm and a bulk density ranging from about 0.3 g/ml to about 0.5 g/ml, or a pore size ranging from about 50 nm to about 150 nm and a bulk density ranging from about 0.3 g/ml to about 0.5 g/ml, although the SCP used in the method according to the invention comprises mesopores, micropores, macropores, or a combination thereof, wherein the mesopores are 2 nm to 50 nm in diameter, the micropores are less than 2 nm in diameter, and the macropores are 50 to 120 nm in diameter. Carbonaceous material may comprise an adsorbent.

Carbonaceous materials of the type disclosed herein may be prepared such that a hot solution (85°C - 90°C) of 100 weight parts of RO-NOVOLAC in 135 weight parts of ethylene glycol was blended with hot solution (85°C - 90°C) of 20 weight parts of hexamine in 135 weight parts of ethylene glycol. The resulting hot resin solution was poured into 2000 weight parts of stirred hot (145°C) mineral oil containing 4 weight parts of the drying oil and formed an emulsion which was formed into beads and further heated. The slurry beads were then separated from the oil and carbonized. The resin beads were carbonized in shallow bed tray in the tube furnace in the flow of carbon dioxide. The temperature was ramped from 20 °C to 800 °C in 200 min and held there for 30 min.

The adsorbent material used in the method according to the invention comprises synthetic carbon particles (SCP) containing micro-, meso- and macropores from porous phenolic resins. As used herein, the term "micropore" refers to a pores with diameter <2 nm, as measured by nitrogen adsorption and mercury porosimetry methods and as defined by IUPAC. As used herein, the term "mesopore" refers to pores with diameter from 2 nm to 50 nm, as measured by nitrogen adsorption and mercury porosimetry methods and as defined by IUPAC. As used herein, the term "macropore" refers to pores with diameters larger than 50 nm, although the macropores used in the method according to the present invention are 50 to 120 nm in diameter, as measured by nitrogen adsorption and mercury porosimetry methods and as defined by IUPAC. There are two types of macropores. In macroporous beads they are located within beads and formed by pore-formers. Their sizes are between 10-1000 nm, between 50-500 nm, and between 70-200 nm. These macropores have previously proved effective in adsorption of cytokines.

A SCP suitable for use in the present disclosure may have any shape compatible with the compositions and methodologies disclosed herein. For example the shape of the SCP may be that of an irregular granule, a low angularity shape, spherical (e.g., bead), pellet, minilith, monolith, etc..... For simplicity, the present disclosure may refer to the use of beads of the SCB however it is to be understood the SCP may be of any suitable shape. The SCPs may be formed using any suitable methodology to results in a material having the properties disclosed herein. In an exemplary method for the formation of an SCP, a precursor resin formulation is used which comprises a large proportion of pore former, e.g. 250 parts ethylene glycol or other pore former to 100 parts of resin-forming components.

The SPC may comprise a bimodal distribution of pores, the pore structure as estimated by nitrogen adsorption porosimetry comprising micropores and mesopores or macropores. The value for the differential of pore volume with respect to the logarithm of pore radius (dV/d log R) for the mesopores is greater than 0.2 for at least some values of pore size in the range 20-500Å. The mesoporous carbon may have a BET surface area of 250-800 m²/g without activation. It may be activated by heating and may then have surface areas of up to 2000 m²/g and even higher e.g. 1000-2000 m²/g.

In some embodiments a SPC may comprise an activated Bet surface area of 300-700 m²/g; in another embodiment a SPC may comprise an activated Bet surface area of 400-650 m²/g; in a further embodiment a SPC may comprise an activated Bet surface area of 450-600 m²/g; and in a still further embodiment a SPC may comprise an activated Bet surface area of 525-575 m²/g.

In some embodiments a SPC may comprise an Bet surface area without activation of 300-700 m2/g; in another embodiment a SPC may comprise Bet surface area without activation of 400-650 m²/g; in a further embodiment a SPC may comprise a Bet surface area without activation of 450-600 m²/g; and in a still further embodiment a SPC may comprise a Bet surface area without activation of 525-575 m²/g.

Therefore, disclosed herein, but not part of the claimed invention, are methods of diagnosing a medical condition in a subject which may comprise obtaining plasma from the whole blood of a subject (wherein for example the subject suffers from a disease state or medical condition such as cancer); quantifying the amount of exosomes in the plasma; and comparing the amount of exosomes in the plasma with a control amount of exosomes (e.g., the amount of exosomes in the plasma of a healthy control subject).

One of ordinary skill, upon diagnosis of a medical condition by the methods disclosed herein may further administer a therapeutic treatment, or a therapeutically effective amount of a suitable compound or medicament described herein but not part of the claimed invention.

A "therapeutically effective amount" is an amount sufficient to effect beneficial or desired results. For example, a therapeutically effective amount is one that achieves the desired therapeutic effect. This amount can be the same or different from a prophylactically effective amount, which is an amount necessary to reduce or inhibit a condition, disease or an infection. An effective amount can be administered in one or more administrations, applications, or dosages. A therapeutically effective amount of a composition depends on the condition and subsequent composition selected. The compositions can be administered from one or more times per day to one or more times per week, including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a known therapeutically effective amount of such compositions can include a single treatment or a series of treatments.

Treatments may comprise suitably formulated compounds and agents, e.g., small molecules, nucleic acids, polypeptides, and antibodies (all of which can be referred to herein as "active compounds"), and can be incorporated into pharmaceutical compositions for administration post diagnosis by the method described herein. Such compositions typically include the active compound suitable for treating the identified medical condition, wherein the above method is used to identify such a condition, and a pharmaceutically acceptable carrier. A "pharmaceutically acceptable carrier" can include solvents, dispersion media, coatings, antibacterial and antifungal agents, isotonic and absorption delaying agents, and the like, compatible with pharmaceutical administration. Supplementary active compounds can also be incorporated into the compositions. The pharmaceutical compositions can be included in a container, pack, or dispenser together with instructions for administration.

Such a treatment that may be administered post diagnosing a condition by the methods described herein, but not part of the claimed invention, may comprise a pharmaceutical composition which is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide.

The disease diagnosed by the methods described herein but not part of the claimed invention may include but are not limited to cancer, an advanced malignancy, amyloidosis, neuroblastoma, meningioma, hemangiopericytoma, multiple brain metastase, glioblastoma multiforms, glioblastoma, brain stem glioma, poor prognosis malignant brain tumor, malignant glioma, anaplastic astrocytoma, anaplastic oligodendroglioma, neuroendocrine tumor, rectal adenocarcinoma, Dukes C & D colorectal cancer, unresectable colorectal carcinoma, metastatic hepatocellular carcinoma, Kaposi's sarcoma, karotype acute myeloblastic leukemia, Hodgkin's lymphoma, non-Hodgkin's lymphoma, cutaneous T-Cell lymphoma, cutaneous B-Cell lymphoma, diffuse large B-Cell lymphoma, low grade follicular lymphoma, malignant melanoma, malignant mesothelioma, malignant pleural effusion mesothelioma syndrome, peritoneal carcinoma, papillary serous carcinoma, gynecologic sarcoma, soft tissue sarcoma, scleroderma, cutaneous vasculitis, Langerhans cell histiocytosis, leiomyosarcoma, fibrodysplasia ossificans progressive, hormone refractory prostate cancer, resected high-risk soft tissue sarcoma, unrescectable hepatocellular carcinoma, Waldenstrom's macroglobulinemia, smoldering myeloma, indolent myeloma, fallopian tube cancer, androgen independent prostate cancer, androgen dependent stage IV non-metastatic prostate cancer, hormone-insensitive prostate cancer, chemotherapy-insensitive prostate cancer, papillary thyroid carcinoma, follicular thyroid carcinoma, medullary thyroid carcinoma, or leiomyoma. Further, a cancer may be advanced malignancy, amyloidosis, locally advanced bladder cancer, metastatic transitional cell bladder cancer, relapsed brain tumor, progressive brain tumor, neuroblastoma, meningioma, hemangiopericytoma, multiple brain metastase, glioblastoma multiforms, glioblastoma, brain stem glioma, poor prognosis malignant brain tumor, malignant glioma, anaplastic astrocytoma, anaplastic oligodendroglioma, metastatic breast cancer, neuroendocrine tumor, rectal adenocarcinoma, Dukes C & D colorectal cancer, unresectable colorectal carcinoma, metastatic hepatocellular carcinoma, Kaposi's sarcoma, karotype acute myeloblastic leukemia, Hodgkin's lymphoma, non-Hodgkin's lymphoma, cutaneous T-Cell lymphoma, cutaneous B-Cell lymphoma, diffuse large B-Cell lymphoma, low grade follicular lymphoma, malignant melanoma, malignant mesothelioma, stage IIIB non-small cell lung cancer, malignant pleural effusion mesothelioma syndrome, multiple myeloma, peritoneal carcinoma, papillary serous carcinoma, gynecologic sarcoma, soft tissue sarcoma, scleroderma, cutaneous vasculitis, Langerhans cell histiocytosis, leiomyosarcoma, fibrodysplasia ossificans progressive, hormone refractory prostate cancer, resected high-risk soft tissue sarcoma, unrescectable hepatocellular carcinoma, Waldenstrom's macroglobulinemia, smoldering myeloma, indolent myeloma, fallopian tube cancer, androgen independent prostate cancer, androgen dependent stage IV non-metastatic prostate cancer, hormone-insensitive prostate cancer, chemotherapy-insensitive prostate cancer, papillary thyroid carcinoma, follicular thyroid carcinoma, medullary thyroid carcinoma, or leiomyoma.

Other diagnosed conditions may include: diabetic retinopathy, retinopathy of prematurity, corneal graft rejection, neovascular glaucoma, retrolental fibroplasia, proliferative vitreoretinopathy, trachoma, myopia, optic pits, epidemnic keratoconjunctivitis, atopic keratitis, superior limbic keratitis, pterygium keratitis sicca, sjogrens, acne rosacea, phylectenulosis, syphilis, lipid degeneration, bacterial ulcer, fungal ulcer, Herpes simplex infection, Herpes zoster infection, protozoan infection, Kaposi sarcoma, Mooren ulcer, Terrien's marginal degeneration, mariginal keratolysis, rheumatoid arthritis, systemic lupus, polyarteritis, trauma, Wegeners sarcoidosis, Scleritis, Steven's Johnson disease, periphigoid radial keratotomy, sickle cell anemia, sarcoid, pseudoxanthoma elasticum, Pagets disease, vein occlusion, artery occlusion, carotid obstructive disease, chronic uveitis, chronic vitritis, Lyme's disease, Eales disease, Behcet's disease, retinitis, choroiditis, presumed ocular histoplasmosis, Bests disease, Stargarts disease, pars planitis, chronic retinal detachment, hyperviscosity syndromes, toxoplasmosis, sclerosing cholangitis, or rubeosis, wherein any such condition may be treated accordingly, once identified.

A method of treating such a medical condition in a subject suffering from such a disease state is described herein but is not part of the claimed invention, wherein the method may comprise removing at least a portion of the exosomes from the whole blood of the subject; and administering the whole blood that was subjected to the exosome removal process back to the patient.

A method of treating a medical condition in a subject described herein but not part of the claimed invention may comprise removing at least a portion of the exosomes from the plasma of the subject and administering the plasma that was subjected to the exosome removal process back to the patient. The removal of exosomes derived from non-healthy cells (e.g., diseased cells) may be removed from the whole blood or plasma of a subject by contacting the whole blood or plasma with an adsorbent material, wherein the disease derived exosomes undergo adsorption onto the adsorbent material. Whole blood (or a component thereof such as plasma) may be contacted with a synthetic carbon particle (SCP) as described in U.S. Patent 9,669,151 to form an exosome-SCP complex. The exosome can be retained onto the SCP via any suitable interactions, such as electrostatic interactions, physical interactions, chemical interactions, and the like, or combinations thereof, in order to form the exosome-SCP complex. The exosome-SCP complex can comprise any suitable type of chemical bonds and/or interactions between the exosome and the SCP, such as covalent bonds, ionic bonds, hydrogen bonds, electrostatic interactions, dipole-dipole interactions, van der Waals forces, and the like, or combinations thereof.

In an embodiment, the exosome-SCP complex can comprise exosomes in an amount of from about 10² exosomes/g SCP to equal to or greater than about 10⁹ exosomes/g SCP, alternatively from about 10³ exosomes/g SCP to about 10⁸ exosomes/g SCP, or alternatively from about 10⁶ exosomes/g SCP to about 10⁸ exosomes/g SCP. The amount of exosomes that binds to SCP to form the exosome-SCP complex from a given volume of whole blood and/or plasma can be indicative of the presence or the absence of a medical condition, such as cancer. Further, the removal of exosomes from whole blood can provide for treating or curing certain medical conditions, such as cancer, for example via the removal of disease derived exosomes from whole blood or plasma of a disease compromised subject, and return of exosome-depleted whole blood (or component thereof such as plasma) to the subject suffering the medical condition.

Methods of treating a subject are disclosed herein but do not form part of the claimed invention, that comprise contacting a bodily fluid (e.g., whole blood, plasma, cerebrospinal fluid) with a SCP of the type disclosed herein.

The subject may thus be diagnosed with a malignant tumor that has not metastasized. The treatment may prevent or delay metastasis of the malignant tumor.

As laid out herein, such a method may then be used to control cancer metastasis alone, or in conjunction with other forms of treatment. Several carbon beads have been identified as being efficient in adsorption of exosomes, whilst in some embodiments SCP-1 was determined to be most efficient.

SCP-1 in some embodiments comprises a carbonized material, wherein in some further embodiments SCP-1 comprises phenolic resins, wherein the phenolic resins are configured to comprise a surface area of about 300 to about 800 m²/g, about 400 to about 600 m²/g, about 500 to about 570 m²/g and about 525 m²/g to about 575 m²/g. In some other embodiments SCP-1 comprises pores, wherein the pores may be: about 2 nm to about 120 nm; about 10 nm to about 100 nm; about 20 nm to about 90 nm, about 25 nm to about 85 nm, about 30 nm to about 80 nm, about 35 nm to about 75nm, about 40 nm to about 70 nm, about 45 nm to about 70 nm, about 50 nm to 65 nm, and about 55 nm to about 60 nm.

In some embodiments SCP-1 is not activated. In some other embodiments the carbon material that comprises SCP-1 is not an activated carbon. In other embodiments SCP-1 is activated. In some other embodiments the carbon material that comprises SCP-1 is activated carbon.

In some embodiments, experiments to assess the efficiency of adsorption of such carbon sources included utilizing plasma from a patient with metastatic ovarian tumor, wherein the plasma comprises tumor derived exosomes.

In further embodiments, experiments were performed on cellular media from glioblastoma cells line and observed a similar efficiency of adsorption as seen for exosomes derived from metastatic ovarian tumors.

Exosomes derived from the plasma of health control subjects were also found to undergo adsorption by such carbon materials or SCPs, but with significantly less efficiency. SPC SCP-1, in some embodiments may therefore be specific for exosomes. This hypothesis is supported because free DNA was not detected or extracted from the carbon material after exposure to plasma or blood. SCP-1 is the selective for exosomes from at least ovarian and glioblastoma-derived exosomes. Also, SCP-1 did not remove all the exosomes present in the subject derived sample suggesting the carbon material would comprise low toxicity.

### EXAMPLES

Experimental Section: Embodiments of the adsorption and binding of plasma molecules and particles to SPCs such as carbon SCP-1 are disclosed herein.

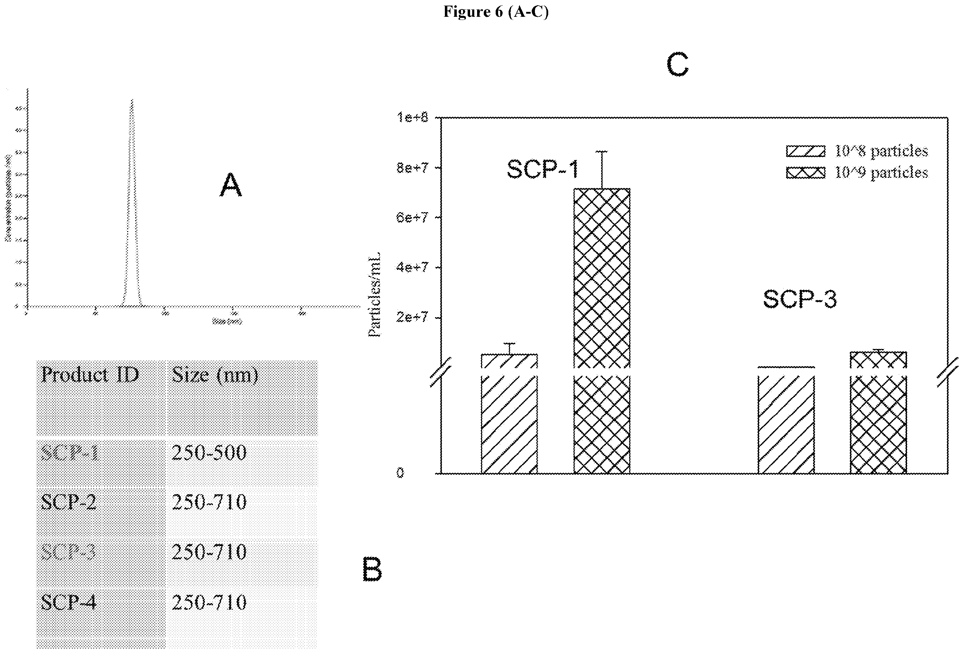

Initial experiments were performed to assess embodiments of a number of adsorbent materials used herein: purified exosomes (50-100 nm in size) and about 10⁸ and 10⁹ in number, were contacted with different SCPs: carbon SCP-1 and SCP-3, and were agitated for about 24 hrs., in order to allow binding/adsorption of the exosomes to the different SCPs. With the addition of an overburden of exosomes (10⁹), SCP-1 was able to remove ~10⁷ exosomes (Figure 6 (A-C). Embodiments described herein, thus reveal that carbon SCP-1 can effectively bind/adsorb exosomes in a greater number than SCP-3.

This mechanism was confirmed for clinical samples using plasma from an ovarian cancer patient as compared to plasma from a healthy donor of a similar age, as described in Experiment 1:

Experiment 1: In one embodiment, exosome adsorption/binding from a comparable plasma volume of an ovarian cancer patient was evaluated before and after surgery, wherein: plasma from said subject was diluted at 1/10 in 1 × PBS and 1 mL of the diluent added to 0.2 g of SCP-1, wherein in some embodiments SCP-1 has a particle size of between 250-500 nm and a pore size of about 27 nm.

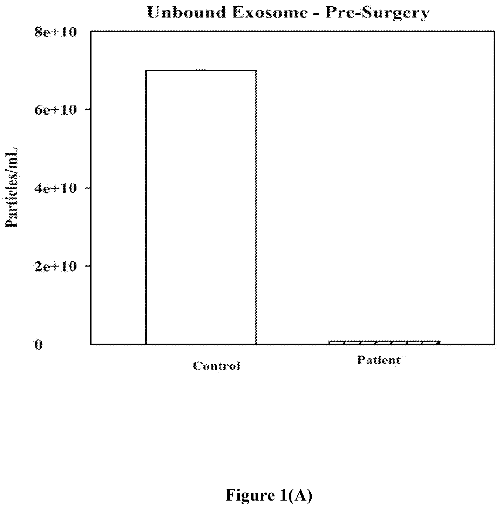

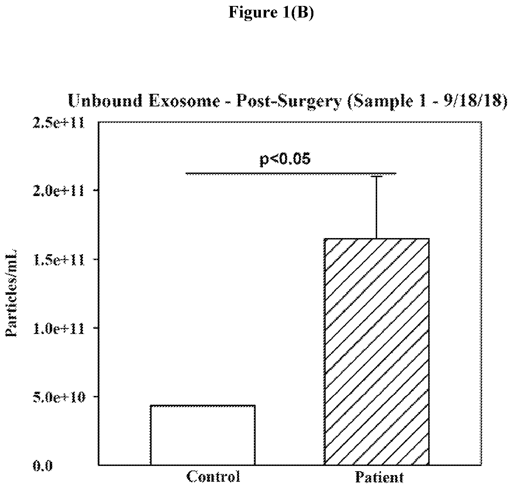

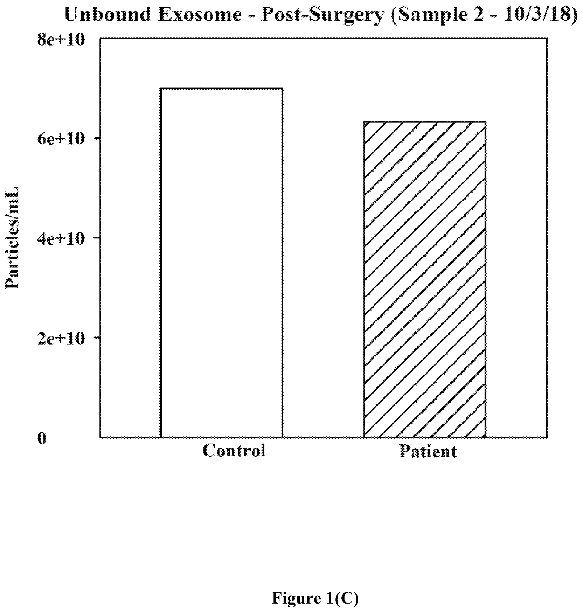

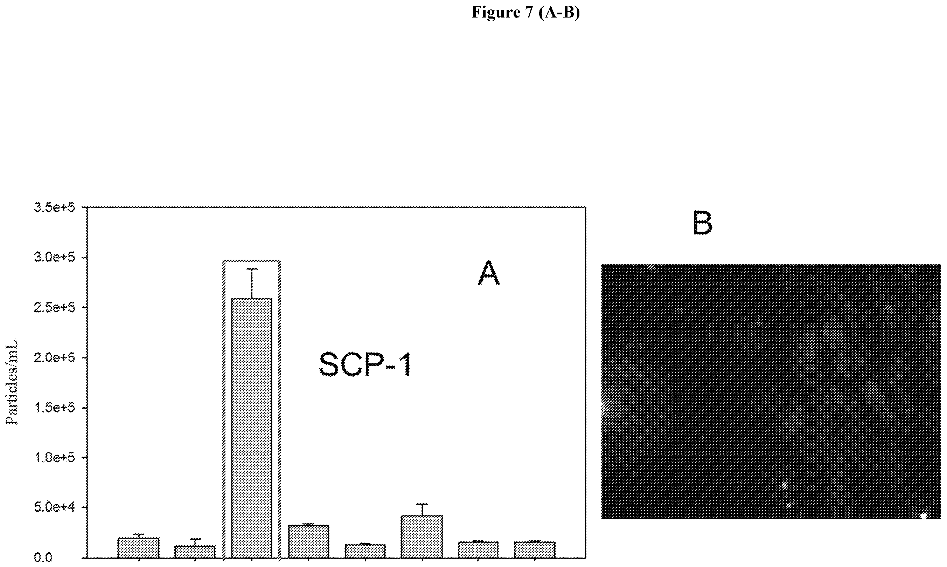

The mixture was incubated at room temperature with continuous mixing on a rotator. After 24 hrs., the mixture was centrifuged at 1500 rpm for 5 mins at room temperature. The supernatant, which contained unbound-exosomes, was transferred to a fresh tube. The pellet was resuspended in 200 µL of 1x PBS and the mixture boiled for 10 min at 95°C to detach the bound exosomes. The sample was centrifuged for 5 mins at 1500 rpm and the supernatant, which contained the bound exosomes, was placed into another tube. The unbound and bound exosomes were analyzed by Nanoparticle Tracking Analysis (NTA) wherein NTA, is a technique used for measuring and analyzing single particle size and concentration (*See* Figure 7 (A) and Figure 7(B)). An experimental control was performed in parallel, wherein the plasma from a healthy, age-match subject was subjected to similar treatment. Pre surgery, the subject's plasma as depicted in Figure 1(a) comprises low numbers of unbound exosomes as compared to the control subject. An experimental control was performed in parallel, wherein the plasma from a healthy, age-match subject was subjected to similar treatment (Figure 1(a)). Thus, in one embodiment, as there were relatively few unbound exosomic particles pre-surgery, as compared to the increased number of tumor-derived exosomes (as seen in Figure 1 (b) and Figure 1(C) for the post operative/surgical plasma sample) there may in fact be an tumor-derived exosomic expression marker that interacts with the carbon (SCP-1).

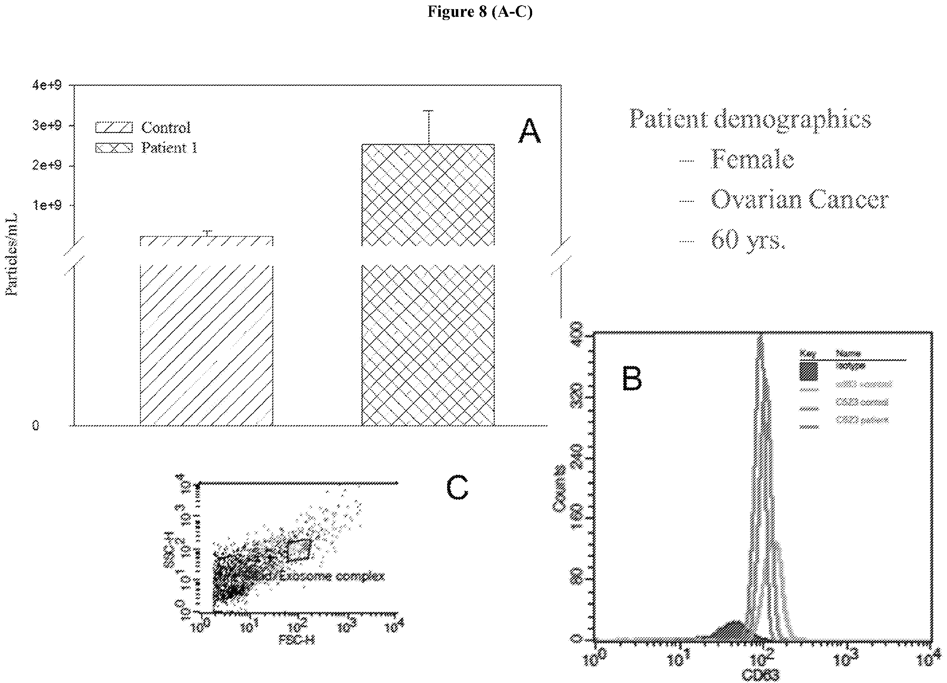

These data indicate that this carbon SCP-1 can be used clinically to reduce overburden of exosomes in ovarian cancer subjects (Figures 8 (A-C). The ability of the exosome to bind the carbon (SCP-1) in some embodiments also appears to normalize with time.

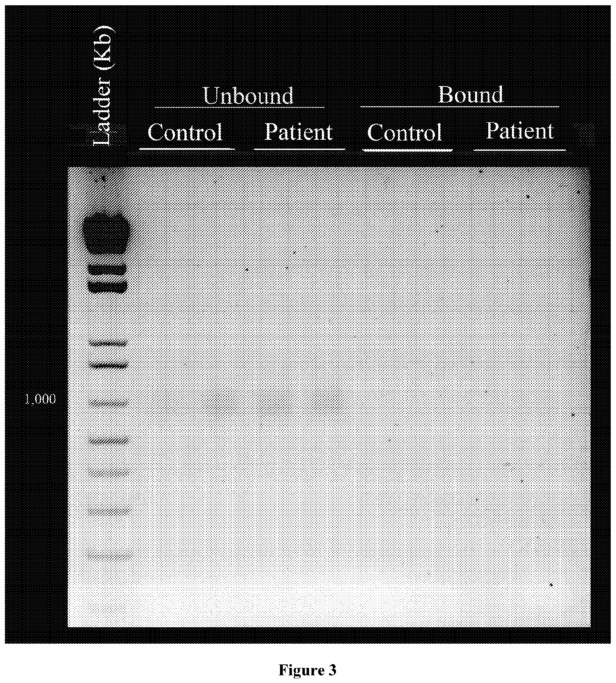

Experiment 2: in some embodiments, studies were performed to determine if DNA can bind to the SCP, such as in some embodiments to SCP-1. The results show in Figure 3, that cell-free DNA is present in the supernatant of experiment 1, but no evidence of bound DNA to the carbon was apparent as indicated by the gel electrophoresis of bound and unbound samples from Experiment 1 as described herein which were electrophoresed on 2% agarose.

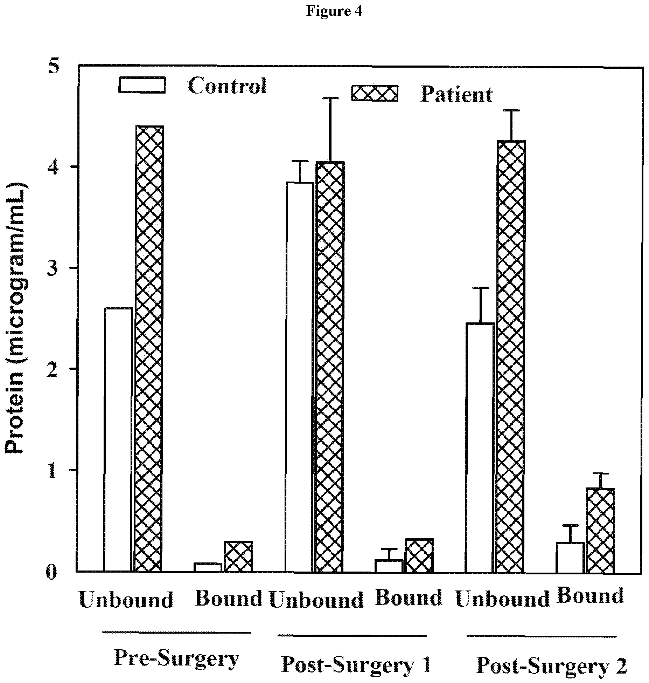

Experiment 3: in some embodiments, the total amount of protein was assessed in the bound and unbound samples from Experiment 1 as compared to the control. The binding/adsorption of protein in the samples from Experiment 1, were assessed by a Bradford Assay for total protein as seen in Figure 4, wherein the pre, and post surgery data-1 and post surgery data-2 for bound and unbound protein from a patient and control where evaluated. In some embodiments, it can be seen that the amount of total protein increases with time post surgery in the bound exosomic samples.

Therefore, disclosed herein, but not part of the claimed invention is a method for identifying and treating an individual or subject suffering from such medical conditions as, but not limited to: septic shock, cancer, glioblastoma multiform (GMB), and preeclampsia, or other such conditions by contacting whole blood or plasma from the individual or subject with an adsorbent material, preferably a SCP, or most preferably SCP-1, and removing in some instances, disease derived or disease inducing exosomes from the plasma or blood of the subject. The exosomes are quantified and compared to controls in order to identify the medical condition, and an appropriate treatment may be administered by one of skill in the art. Described herein, but not part of the claimed invention, the treated blood may be reconstituted and administered back to the subject in order to treat such a medical condition, herein the treated blood may be administered alone, or in combination with any other therapy or therapeutic amount of pharmaceutical composition by one of ordinary skill in the art.

Where numerical ranges or limitations are expressly stated, such express ranges or limitations should be understood to include iterative ranges or limitations of like magnitude falling within the expressly stated ranges or limitations (e.g., from about 1 to about 10 includes, 2, 3, 4, etc.; greater than 0.10 includes 0.11, 0.12, 0.13, etc.). For example, whenever a numerical range with a lower limit, R_{L}, and an upper limit, R_{U}, is disclosed, any number falling within the range is specifically disclosed. In particular, the following numbers within the range are specifically disclosed: R=R_{L} +k* (R_{U}-R_{L}), wherein k is a variable ranging from 1 percent to 100 percent with a 1 percent increment, i.e., k is 1 percent, 2 percent, 3 percent, 4 percent, 5 percent, ....., 50 percent, 51 percent, 52 percent, ....., 95 percent, 96 percent, 97 percent, 98 percent, 99 percent, or 100 percent. Moreover, any numerical range defined by two R numbers as defined in the above is also specifically disclosed. When a feature is described as "optional," both embodiments with this feature and embodiments without this feature are disclosed. Similarly, the present disclosure contemplates embodiments where this feature is required and embodiments where this feature is specifically excluded. Both alternatives are intended to be within the scope of the claim. Use of broader terms such as comprises, includes, having, etc. should be understood to provide support for narrower terms such as consisting of, consisting essentially of, comprised substantially of, etc.

## Claims

1. A method for quantifying an amount of exosomes in whole blood comprising:
obtaining whole blood from a subject;
separating the whole blood into plasma, and at least one cellular blood fraction, wherein the plasma comprises a first amount of unbound exosomes;
contacting the plasma with an adsorbent material comprising a synthetic carbon particle (SCP), and forming treated plasma, and a complex, wherein the treated plasma comprises a second amount of unbound exosomes, and the complex comprises an amount of SCP-bound-exosomes; and
comparing the second amount of unbound exosomes with a first control and/or comparing the SCP-bound-exosomes with a second control;
wherein the SCP comprises mesopores, micropores, macropores, or a combination thereof, further wherein the mesopores are 2 nm to 50 nm in diameter, wherein the micropores are less than 2 nm in diameter, and wherein the macropores are 50 to 120 nm in diameter.

2. The method of claim 1, wherein the first control comprises a known amount of unbound exosomes, and the second control comprises a known amount of bound-exosomes.

3. The method of claim 1, wherein the subject is diagnosed with or suspected of having a medical condition when the second amount of unbound exosomes are different from the first control and/or when the amount of SCP-bound-exosomes are different from the second control.

4. The method of claim 1, wherein the SCP comprises a surface area of 300-900 m²/g.

## Patentansprüche

1. Verfahren zur Quantifizierung einer Menge von Exosomen in Vollblut, umfassend:
Erhalten von Vollblut von einem Individuum;
Trennen des Vollbluts in Plasma und wenigstens eine zelluläre Blutfraktion, wobei das Plasma eine erste Menge ungebundener Exosomen umfasst;
In-Kontakt-Bringen des Plasmas mit einem Adsorbiermaterial, das ein synthetisches Kohlenstoffpartikel (Synthetic Carbon Particle, SCP) umfasst, und Bildung von behandeltem Plasma und einem Komplex, wobei das behandelte Plasma eine zweite Menge ungebundener Exosomen und der Komplex eine Menge SCPgebundener Exosomen umfasst; und
Vergleichen der zweiten Menge ungebundener Exosomen mit einer ersten Kontrolle und/oder Vergleichen der SCPgebundenen Exosomen mit einer zweiten Kontrolle;
wobei das SCP Mesoporen, Mikroporen, Makroporen oder eine Kombination davon umfasst, ferner wobei die Mesoporen einen Durchmesser von 2 nm bis 50 nm aufweisen, wobei die Mikroporen einen Durchmesser von weniger als 2 nm aufweisen und wobei die Makroporen einen Durchmesser von 50 bis 120 nm aufweisen.

2. Verfahren nach Anspruch 1, wobei die erste Kontrolle eine bekannte Menge ungebundener Exosomen und die zweite Kontrolle eine bekannte Menge gebundener Exosomen umfasst.

3. Verfahren nach Anspruch 1, wobei bei dem Individuum ein Leiden diagnostiziert oder vermutet wird, wenn die zweite Menge ungebundener Exosomen von der ersten Kontrolle verschieden ist und/oder wenn die Menge SCPgebundener Exosomen von der zweiten Kontrolle verschieden ist.

4. Verfahren nach Anspruch 1, wobei das SCP eine Oberfläche von 300-900 m²/g umfasst.

## Revendications

1. Procédé de quantification d'une quantité d'exosomes dans du sang total comprenant :
l'obtention de sang total à partir d'un sujet ;
la séparation du sang total en plasma et au moins une fraction de sang cellulaire, le plasma comprenant une première quantité d'exosomes non liés ;
la mise en contact du plasma avec un matériau adsorbant comprenant des particules de carbone synthétique (SCP) et la formation de plasma traité et d'un complexe, le plasma traité comprenant une seconde quantité d'exosomes non liés et le complexe comprenant une quantité d'exosomes liés aux SCP ; et
la comparaison de la seconde quantité d'exosomes non liés avec un premier témoin et/ou la comparaison des exosomes liés aux SCP avec un second témoin ;
dans lequel les SCP comprennent des mésopores, des micropores, des macropores ou une combinaison de ceux-ci, dans lequel en outre les mésopores ont un diamètre de 2 nm à 50 nm, dans lequel les micropores ont un diamètre inférieur à 2 nm et dans lequel les macropores ont un diamètre de 50 à 120 nm.

2. Procédé selon la revendication 1, dans lequel le premier témoin comprend une quantité connue d'exosomes non liés et le second témoin comprend une quantité connue d'exosomes liés.

3. Procédé selon la revendication 1, dans lequel le sujet est diagnostiqué comme ayant ou étant susceptible d'avoir une affection médicale lorsque la seconde quantité d'exosomes non liés est différente de celle du premier témoin et/ou lorsque la quantité d'exosomes liés aux SCP est différente de celle du second témoin.

4. Procédé selon la revendication 1, dans lequel les SCP comprennent une surface spécifique de 300 à 900 m²/g.
